# DEMANDE DE BREVET EUROPEEN

(11) **EP 2 810 676 A1**
(43) Date de publication de la demande: **10.12.2014**
(21) Numéro de dépôt: 14171090.5
(22) Date de dépôt: 04.06.2014
(51) Int. Cl.: A61M 16/00

(54) **Dispositif de suivi à distance d'au moins un dispositif médical**

(30) Priorité: 05.06.2013 FR 1355152
(71) Demandeur: L.3 Medical, 38070 Saint Quentin Fallavier (FR)
(72) Inventeur: Fernandes, Philippe, 38118 SAINT BAUDILLE DE LA TOUR (FR)
(74) Mandataire: Myon, Gérard Jean-Pierre

(57) **Abrégé**

L'invention concerne un dispositif (9) de suivi à distance d'au moins un dispositif médical (7) comportant au moins une partie (11) animée de vibrations lorsque le dispositif médical (7) est utilisé par un patient pour recevoir un traitement médical. Le dispositif de suivi (9) comprend : au moins un capteur (15) destiné à être en contact mécanique avec la partie (11) et adapté pour détecter les vibrations et pour produire un signal primaire (23) électrique ou hertzien représentatif des vibrations, et une unité de traitement local (13) adaptée pour recevoir le signal primaire (23), pour extraire du signal primaire (23) au moins une information représentative d'une durée d'utilisation du dispositif médical (7) par le patient, et pour produire au moins un signal secondaire (29) contenant ladite information, le signal secondaire (29) étant destiné à être envoyé à au moins un serveur distant (3) adapté pour recevoir le signal secondaire (29) et pour extraire ladite information du signal secondaire (29). L'invention concerne également un ensemble (5) comprenant au moins un dispositif médical (7) et un tel dispositif de suivi (9), ainsi qu'une installation (1) comportant au moins un tel ensemble (5) et au moins le serveur distant.

## Description

La présente invention concerne un dispositif de suivi à distance d'au moins un dispositif médical comportant au moins une partie animée de vibrations lorsque le dispositif médical est utilisé par un patient pour recevoir un traitement médical.

L'invention concerne aussi un ensemble intégrant au moins un dispositif médical et au moins un tel dispositif de suivi. L'invention concerne enfin une installation comprenant au moins un tel ensemble et au moins un serveur distant.

Actuellement, de plus en plus de traitements médicaux mettent en oeuvre des dispositifs médicaux utilisés à domicile par un patient. Il s'agit par exemple de concentrateurs, cuve, portable, bouteille d'oxygène pour le traitement d'affections respiratoires, d'appareils à pression positive continue pour le traitement de l'apnée du sommeil, de ventilateurs à deux niveaux de pression pour le traitement de la broncho-pneumopathie chronique obstructive (ou BPCO), de pompes à nutrition, nébuliseur, aspirateur de mucosités ou de pousse seringues et tout autre dispositif médical. De tels dispositifs médicaux, lorsqu'ils sont en fonctionnement, créent des vibrations, par exemple d'un boîtier, d'un châssis ou d'un organe interne.

L'avantage de tels dispositifs médicaux est de permettre au patient de recevoir le traitement médical chez lui ou dans le lieu dans lequel il se trouve. Le patient n'a pas besoin de se rendre chez un médecin ou à l'hôpital, ce qui permet également de réduire le coût du traitement médical. La question se pose toutefois de contrôler l'observance du traitement médical par le patient, pour des raisons médicales ou légales.

Pour résoudre ce problème, il est connu de contrôler la mise en tension du dispositif médical. A cette fin le dispositif médical est par exemple doté d'un dispositif de suivi pour enregistrer les durées des périodes de mise en tension du dispositif médical. Les résultats sont avantageusement relevés à intervalles de temps réguliers, par exemple par une personne chargée de la maintenance du dispositif, ou une personne soignante, en général directement sur le dispositif médical par simple lecture.

Toutefois, un tel suivi détecte simplement le fait que le dispositif médical soit mis en tension, et non le fait que ce dernier soit effectivement utilisé par le patient pour recevoir le traitement médical. Les résultats relevés sont éventuellement faussés dès lors que le patient oublie de mettre le dispositif médical hors tension après usage, ou bien met le dispositif médical en tension sans pour autant suivre le traitement médical.

Un but de l'invention est de fournir un dispositif de suivi à distance d'au moins un dispositif médical fournissant notamment une information plus pertinente concernant l'observance du traitement médical par le patient.

A cet effet, l'invention a pour objet un dispositif de suivi à distance d'au moins un dispositif médical comportant au moins une partie animée de vibrations lorsque le dispositif médical est utilisé par un patient pour recevoir un traitement médical, le dispositif de suivi comprenant :
- au moins un capteur destiné à être en contact mécanique avec la partie et adapté pour détecter les vibrations et pour produire un signal primaire électrique ou hertzien représentatif des vibrations, et
- une unité de traitement local adaptée pour recevoir le signal primaire, pour extraire du signal primaire au moins une information représentative d'une durée d'utilisation du dispositif médical par le patient, et pour produire au moins un signal secondaire contenant ladite information, le signal secondaire étant destiné à être envoyé à au moins un serveur distant adapté pour recevoir le signal secondaire et pour extraire ladite information du signal secondaire.

Selon des modes particuliers de réalisation, le dispositif de suivi comprend l'une ou plusieurs des caractéristiques suivantes, prise(s) isolément ou selon toutes les combinaisons techniquement possibles :
- le capteur comprend au moins une jauge à effet piézoélectrique adaptée pour transformer les vibrations en une tension électrique ;
- le dispositif de suivi comprend en outre au moins une liaison filaire reliant le capteur à l'unité de traitement local et adaptée pour véhiculer le signal primaire du capteur vers l'unité de traitement local ;
- la liaison filaire est adaptée pour alimenter le capteur en énergie électrique en provenance de l'unité de traitement local ;
- l'unité de traitement local comprend un émetteur hertzien destiné à émettre le signal secondaire vers le serveur distant ;
- le capteur comprend un émetteur hertzien adapté pour émettre le signal primaire vers l'unité de traitement local, et l'unité de traitement local comprend un récepteur hertzien pour recevoir le signal primaire ;
- le capteur est adapté pour détecter uniquement des vibrations de la partie dont l'intensité dépasse un seuil de détection ajustable ;
- l'unité de traitement local est une tablette ou un téléphone mobile évolué, également connu sous la dénomination de « *smartphone » ;*
- l'unité de traitement local est adaptée pour communiquer avec le serveur distant par un protocole GSM, GPRS ou UMTS ;
- l'unité de traitement local est adaptée pour communiquer à distance avec le serveur distant par un réseau analogique ou par fibres optiques ;
- l'unité de traitement local est avantageusement adaptée pour communiquer sans fil avec le capteur, par un protocole Wi-Fi ou Bluetooth.

L'invention concerne aussi un ensemble comprenant :
- au moins un dispositif médical comportant au moins une partie animée de vibrations lorsque le dispositif médical est utilisé par un patient pour recevoir un traitement médical, et
- un dispositif de suivi tel que mentionné ci-dessus, le capteur étant en contact mécanique avec la partie du dispositif médical.

Selon des modes particuliers de réalisation, l'ensemble comprend l'une ou plusieurs des caractéristiques suivantes, prise(s) isolément ou selon toutes les combinaisons techniquement possibles :
- le capteur est fixé sur une surface de réception de la partie ;
- l'ensemble comprend au moins un ruban adhésif collé, d'une part, sur le capteur et, d'autre part, sur la surface de réception de part et d'autre du capteur.

L'invention concerne aussi une installation comportant au moins un ensemble tel que mentionné ci-dessus, et au moins un serveur distant adapté pour recevoir le signal secondaire et extraire ladite information du signal secondaire, le signal secondaire étant envoyé au serveur distant.

L'invention sera mieux comprise à la lecture de la description qui va suivre, donnée uniquement à titre d'exemple, et faite en se référant aux dessin annexéx, sur lesquels :
- la figure 1 est une vue représentant schématiquement une installation selon l'invention ;
- la figure 2 est une vue en perspective représentant schématiquement un dispositif selon un mode de réalisation particulier de l'invention ; et
- la figure 3 représente une interface graphique permettant notamment de configurer le dispositif représenté sur la figure 2.

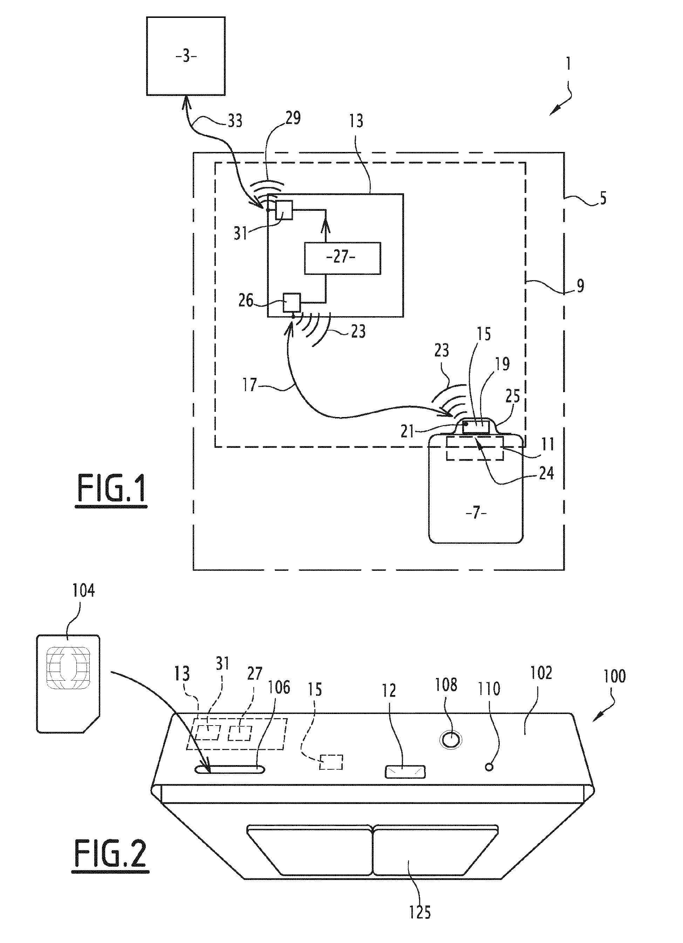

En référence à la figure 1, on décrit une installation 1 comprenant un serveur distant 3 et un ensemble 5.

L'ensemble 5 est par exemple situé chez un patient (non représenté). L'ensemble 5 est situé à une certaine distance, par exemple à au moins quelques dizaines de mètres et plus généralement à au moins quelques kilomètres, du serveur distant 3.

L'ensemble 5 comprend un dispositif médical 7 adapté pour administrer un traitement médical au patient, et un dispositif 9 de suivi à distance du dispositif médical 7.

Le dispositif médical 7 est par exemple un concentrateur, bouteille, cuve ou portable d'oxygène pour le traitement d'affections respiratoires, un dispositif à pression positive continue pour le traitement de l'apnée du sommeil, un ventilateur à deux niveaux de pression pour le traitement de la broncho-pneumopathie chronique obstructive, une pompe à nutrition, ou un pousse seringue...

Le dispositif médical 7 est par exemple situé dans une pièce (non représentée) chez le patient ou dans un lieu donné où se trouve le patient.

Selon une variante non représentée, le dispositif médical 7 est embarqué sur un chariot (non représenté) adapté pour suivre le patient dans ses déplacements. Selon une autre variante non représentée, le dispositif médical 7 est porté directement par le patient, par exemple en bandoulière.

Le dispositif médical 7 comporte une partie 11 qui vibre lorsque le dispositif médical administre effectivement le traitement médical au patient.

La partie 11 est le siège de vibrations dues au fonctionnement du dispositif médical 7. Les vibrations ne naissent pas forcément dans la partie 11. Dans l'exemple représenté, la partie 11 est une portion d'un boîtier du dispositif médical 7. Les vibrations sont créées dans une autre partie (non représentée) du dispositif médical 7, par exemple un moteur ou, plus généralement, une source motrice. Les vibrations se propagent mécaniquement jusqu'à la partie 11.

Selon une autre variante (non représentée), la partie 11 est un tuyau sortant ou entrant du dispositif médical 7. Dans le cas où le dispositif médical 7 délivre de l'air ou de l'air enrichi en oxygène, la partie 11 est par exemple un départ de tuyau conduisant l'air ou l'air enrichi jusqu'au patient.

En variante (non représentée), la partie 11 est une partie d'un châssis du dispositif médical, ou encore un organe interne du dispositif médical, tel qu'un carter de moteur.

Le dispositif de suivi 9 comprend une unité de traitement local 13, et un capteur 15 en contact mécanique avec la partie 11. Le dispositif de suivi 9 comprend en outre avantageusement une liaison filaire 17 reliant le capteur 15 à l'unité de traitement local 13.

Le capteur 15 est adapté pour détecter des vibrations de la partie 11 créées par le fonctionnement du dispositif médical 7 pendant l'administration du traitement médical au patient. Le capteur 15 comprend par exemple une jauge 19 à effet piézoélectrique, connue en soi, et un émetteur 21 pour produire un signal primaire 23 électrique ou hertzien représentatif des vibrations.

Le capteur 15 est par exemple réglable pour détecter uniquement les vibrations de la partie 11 dont l'intensité dépasse un seuil de détection ajustable.

Le capteur 15 est avantageusement alimenté électriquement par la liaison filaire 17 à partir de l'unité de traitement local 13.

Selon une variante non représentée, le capteur 15 est alimenté électriquement par à partir du dispositif médical 7.

Le capteur 15 est avantageusement fixé sur une surface de réception 24 appartenant à la partie 11, par exemple à l'aide d'un ruban adhésif 25.

Dans l'exemple représenté, la surface de réception 24 est une surface externe du boîtier du dispositif médical 7.

Selon un autre mode de réalisation non représenté, la surface de réception 24 est une surface interne du dispositif médical 7.

Le ruban adhésif 25 est fixé sur le capteur 15 et sur la surface de réception 24, de part et d'autre du capteur 15.

Le signal primaire 23 est hertzien dans l'exemple représenté. En variante, le signal primaire 23 est électrique et emprunte la liaison filaire 17.

L'unité de traitement local 13 est située à proximité du dispositif médical 7. Le terme « local » s'entend par opposition au terme « distant ». Par « à proximité » ou « local », on entend avantageusement « dans un rayon de quelques dizaines de mètres au maximum du dispositif médical 7 ».

L'unité de traitement local 13 comprend un récepteur 26 pour recevoir le signal primaire 23, et un module de traitement 27 pour extraire du signal primaire 23 au moins une information représentative d'une durée d'utilisation du dispositif médical 7 par le patient, et pour produire au moins un signal secondaire 29 contenant ladite information. L'unité de traitement local 13 comprend en outre un émetteur 31 pour envoyer le signal secondaire 29 au serveur distant 3.

L'unité de traitement local 13 est avantageusement adaptée pour afficher des informations. Par exemple, l'unité de traitement local 13 comprend une LED indiquant un bon fonctionnement du dispositif de suivi 9 ou un écran pour afficher des informations représentatives du traitement médical suivi par le patient et/ou du bon fonctionnement du dispositif médical 7.

L'unité de traitement local 13 est par exemple une tablette ou un téléphone mobile évolué, également connu sous la dénomination de « *smartphone ».* L'unité de traitement local 13 est avantageusement adaptée pour communiquer avec le serveur distant 3 par un protocole GSM, GPRS ou UMTS.

En variante, l'unité de traitement local 13 est adaptée pour communiquer à distance avec le serveur distant 3 par un réseau 33 analogique ou par fibres optiques.

L'unité de traitement local 13 est avantageusement adaptée pour communiquer localement sans fil avec le capteur 15, par exemple par un protocole Wi-Fi, Bluetooth ou leurs équivalents connus en soi.

La liaison filaire 17 est adaptée pour véhiculer le signal primaire 23 lorsque celui-ci est de nature électrique. Avantageusement, la liaison filaire 17 est adaptée pour alimenter électriquement le capteur 15 à partir de l'unité de traitement local 13.

Le serveur distant 3 apte à recevoir le signal secondaire 29 et à en extraire l'information représentative d'une durée d'utilisation du dispositif médical 7.

Le fonctionnement de l'installation 1 va maintenant être décrit.

Lorsque le dispositif médical 7 n'est pas en fonctionnement, il ne produit pas de vibrations ou bien produit des vibrations dont l'intensité ne dépasse pas le seuil de détection du capteur 15. Ainsi, le signal primaire 23 n'est pas représentatif des durées de non fonctionnement du dispositif médical 7. Les durées correspondantes ne sont pas comptabilisées par le dispositif de suivi 9.

Lorsque le dispositif médical 7 est utilisé par le patient pour l'administration du traitement médical, la partie 11 est animée de vibrations mécaniques qui sont détectées par la jauge 19 et transformées en tension électrique.

A partir de la tension électrique délivrée par la jauge 19, le capteur 15 produit le signal primaire 23 représentatif des vibrations détectées.

Le signal primaire 23 est envoyé vers l'unité de traitement local 13 soit par l'émetteur 21, soit par la liaison filaire 17. La liaison filaire 17 alimente en énergie le capteur 15 pour le fonctionnement de ce dernier.

Le signal primaire 23 envoyé par l'émetteur 21 du capteur 15 est reçu par le récepteur 26 de l'unité de traitement local 13 et envoyé au module de traitement 27. Le module de traitement 27 traite le signal primaire 23 et calcule une durée d'utilisation du dispositif médical 7 par le patient, qui est la durée pendant laquelle le signal primaire 23 indique que des vibrations sont détectées.

Le traitement par le module de traitement 27 comporte par exemple un filtrage et un comptage de temps. Avantageusement, le module de traitement 27 effectue des comparaisons des signaux traités avec des seuils prédéterminés, tels une emprunte vibratoire. De manière encore plus avantageuse, le module de traitement 27 effectue des traitements plus sophistiqués pour identifier dans le signal primaire 23 des perturbations des vibrations dues à l'influence du patient sur le dispositif médical 7. Ces traitements sophistiqués sont connus en eux-mêmes et résultent par exemple d'un calibrage du dispositif médical 7.

Le module de traitement 27 produit un signal secondaire 29 contenant au moins l'information représentative de la durée d'utilisation du dispositif médical 7 par le patient.

Selon un mode de réalisation particulier, le signal secondaire 29 contient d'autres informations relatives représentatives de la qualité du traitement médical suivi par le patient et/ou le diagnostic du bon fonctionnement du dispositif médical 7.

L'émetteur 31 émet le signal secondaire 29 à destination du serveur distant 3.

Le serveur distant 3 reçoit le signal secondaire 29 et le traite pour extraire au moins l'information représentative de la durée d'utilisation du dispositif médical 7 par le patient. Par exemple, l'information extraite est la durée d'utilisation du dispositif médical 7 par le patient dans un intervalle de temps. L'intervalle de temps est par exemple une tranche de 24 heures à partir de midi.

Ainsi, l'information extraite est par exemple la suivante : « durant la tranche de vingt-quatre heures correspondant à telle date, le dispositif médical 7 a été utilisé pendant huit heures ». Ceci permet par exemple de valider que le patient a bien suivi par exemple un traitement de l'apnée du sommeil pendant la tranche de vingt-quatre heures considérée.

Grâce aux caractéristiques décrites ci-dessus, en particulier le capteur 15 et l'unité de traitement local 13, l'information extraite du signal secondaire par le serveur distant 3 représente les durées pendant lesquelles la partie 11 du dispositif médical 7 est animée de vibrations caractéristiques de l'utilisation effective du dispositif médical 7 par le patient et non simplement de la mise en tension du dispositif médical 7. L'information est donc plus pertinente concernant l'observance du traitement médical par le patient.

La caractéristique optionnelle selon laquelle le capteur 15 possède un seuil de détection des vibrations ajustable en intensité permet de régler le capteur 15 de manière à ne pas comptabiliser des périodes de temps pendant lesquelles la partie 11 serait animée de vibrations d'intensité trop faible non liées à l'administration du traitement médical par le dispositif médical 7.

Selon une variante du dispositif de suivi 9, l'unité de traitement local 13 est adaptée pour comparer le signal primaire 23 à un seuil ajustable. Une telle variante permet d'introduire un seuil de détection par le calcul et est avantageuse notamment dans le cas où le capteur 15 n'est pas réglable en seuil de détection.

En référence à figure 2, on décrit un dispositif 100 représentant un mode de réalisation particulier de l'invention.

Le dispositif 100 est analogue au dispositif 9 représenté sur la figure 1. Le dispositif 100 est un dispositif de suivi à distance d'un dispositif médical 7, par exemple un PPC, c'est-à-dire un appareil à pression positive continue.

Les PPC sont utilisés en particulier pour le traitement de l'apnée du sommeil.

Les éléments similaires du dispositif 100 sont désignés sur la figure 2 par les mêmes références numériques et ne seront pas décrits à nouveau. Seules les différences seront décrites en détail ci-après.

Le dispositif 100 diffère du dispositif 9 représenté sur la figure 1 notamment par le fait que le capteur 15 et l'unité de traitement local 13 sont intégrés dans un boîtier 102, par exemple de forme parallélépipédique.

Le dispositif 100 comprend en outre une carte SIM 104, un logement 106 propre à recevoir la carte SIM, un bouton 108 de mise en sommeil ou d'activation du dispositif 100, un voyant d'état 110, une prise de connexion 112, et un organe de fixation 125 adapté pour fixer le dispositif 100 sur le dispositif médical.

Le dispositif 100 est par exemple alimenté électriquement par des piles conventionnelles AAA et possède avantageusement une autonomie supérieure à 500 jours.

Le dispositif 100 comprend par exemple un lecteur de carte de type *push-pull* (en français : pousser-tirer) non représenté.

Le capteur 15 est par exemple adapté pour mesurer les vibrations selon trois directions d'espace arbitraires, avantageusement perpendiculaires entre elles. Par exemple le capteur 15 comprend trois jauges à effet piézoélectrique (non représentées). Le capteur 15 produit trois signaux représentatifs des vibrations du dispositif médical 7 dans les trois directions d'espace.

Le module de traitement 27 comprend avantageusement une mémoire adaptée pour stocker le signal primaire 23 ou une information représentative des vibrations mesurées par le capteur 15.

Le bouton 108 permet de mettre en marche et avantageusement d'éteindre ou de mettre en sommeil le dispositif 100.

La carte SIM 104 permet la connexion du dispositif 100 au serveur distant 3, par exemple via un réseau GPRS. Pour retirer la carte SIM 104, un utilisateur appuie sur la carte SIM 104 et celle-ci sort du lecteur.

L'organe de fixation 125 est par exemple une bande adhésive de type permanent. L'organe de fixation 125 est avantageusement conçu pour résister à une force d'arrachement d'environ 200 N, c'est-à-dire correspondant à environ 20 kg. L'organe de fixation 125 permet avantageusement de décoller le dispositif 100 du dispositif médical 7 sans laisser de traces.

Le voyant d'état 110 comprend par exemple une diode électroluminescente ou LED.

La prise de connexion 112 est par exemple une prise USB (en français BUS, pour bus universel en série).

Pour activer le dispositif 100, un utilisateur introduit la carte SIM 104 dans le lecteur du dispositif. Avant, ou après, l'insertion de la carte SIM 104, l'utilisateur écrit et envoie par exemple un message de type texto (ou SMS en anglais, pour *Short Message Service*) mentionnant une information relative la carte SIM 104. Puis l'utilisateur appuie sur le bouton d'activation 108. Le dispositif 100 se connecte au réseau et utilise le voyant d'état 110 pour signifier à l'utilisateur que la connexion est opérationnelle. Par exemple, le voyant d'état 110 clignote trois fois rapidement.

Grâce à la prise de connexion 112, il est possible de relier le dispositif 100 à un ordinateur (non représenté) comportant un logiciel approprié afin de :
- récupérer le signal primaire 23 ou une information représentative des vibrations mesurées par le capteur 15,
- modifier ou supprimer des données dans la mémoire du module de traitement 27, et/ou
- de visualiser et de modifier des réglages du dispositif 100, avantageusement en temps réel.

Les réglages concernent notamment les traitements mathématiques du signal primaire 23 réalisés par le module de traitement 27 en vue de produire le signal secondaire 29.

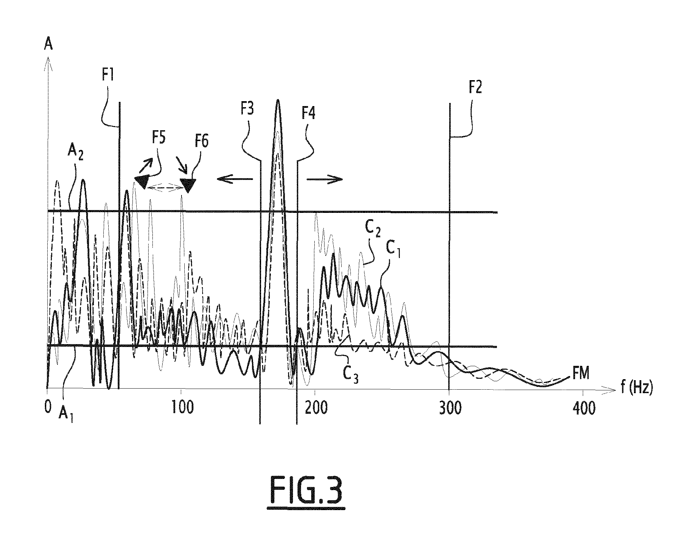

Le logiciel est avantageusement adapté pour permettre une visualisation sur un écran de l'ordinateur connecté au dispositif 100. La visualisation prend par exemple la forme d'un graphique tel que représenté sur la figure 3.

Le module de traitement 27 est par exemple adapté pour réaliser une analyse fréquentielle des signaux fournis par le capteur 15. Dans l'exemple, le traitement comprend un calcul de transformées de Fourier des signaux fournis par le capteur 15 sur des périodes de temps successives de par exemple environ 17 secondes.

Le graphique montre trois courbes C1, C2, C3 représentant les transformées de Fourier obtenues pour l'une des périodes de temps. Le graphique comporte en abscisse la fréquence f, par exemple de 0 à 400 Hz, et en ordonnée le poids spécifique A des harmoniques de chaque fréquence f.

Le logiciel est avantageusement adapté pour sélectionner une plage de fréquences f à considérer à l'aide de curseurs virtuels F1, F2, chacun étant déplaçable vers la gauche ou vers la droite. Seules les portions des courbes C1, C2, C3 situées entre les curseurs virtuels F1 et F2 sont prises en considération pour estimer si le dispositif médical 7 était utilisé pendant la période de temps considérée.

Le logiciel est en outre avantageusement adapté pour sélectionner une plage de poids spécifiques A à considérer à l'aide de curseurs virtuels A1, A2, chacun étant déplaçables vers le haut ou vers le bas. Seules les portions des courbes C1, C2, C3 situées entre les curseurs virtuels A1 et A2 sont prises en considération pour estimer si le dispositif médical 7 était utilisé pendant la période de temps considérée.

Le logiciel est aussi avantageusement adapté pour définir un critère d'exclusion de pics « trop larges » des courbes C1, C2, C3. Par exemple, à l'aide de curseurs virtuels F3, F4, chacun étant déplaçables vers la gauche ou vers la droite, on définit une largeur maximale pour les pics à prendre en considération. En effet, de tels pics « trop larges » correspondent en général à des vibrations ou bruits parasites dans le signal primaire 23.

Enfin, le logiciel permet, par exemple à l'aide de curseurs virtuels F5, F6, de définir des décalages en fréquence à rechercher dans les courbes C1, C2, C3. En effet, de tels décalages sont, pour certains dispositifs médicaux tels que le PPC, représentatifs de l'effet de la respiration du patient sur le dispositif médical 7. Ils sont donc particulièrement représentatifs de l'utilisation du dispositif médical 7.

Le logiciel permet aussi avantageusement de paramétrer le nombre de fréquences d'acquisition à comptabiliser par le module de traitement 27 pour déterminer si le dispositif médical 7 était utilisé pendant la période de temps considérée.

Les valeurs des curseurs F1, F2, F3, F4, A1, A2 et le nombre de fréquences d'acquisition à comptabiliser sont stockés, le cas échéant, dans la mémoire du module de traitement 27.

Le module de traitement 27 est propre à sommer les périodes de temps pour lesquelles une utilisation a été détectée, les périodes de temps appartenant à un intervalle de temps donné. Ainsi, le module de traitement 27 fournit une durée d'utilisation cumulée pendant l'intervalle de temps donné. L'intervalle de temps est par exemple de 24 heures.

## Revendications

1. Dispositif (9; 100) de suivi à distance d'au moins un dispositif médical (7) comportant au moins une partie (11) animée de vibrations lorsque le dispositif médical (7) est utilisé par un patient pour recevoir un traitement médical, le dispositif de suivi (9) comprenant :
- au moins un capteur (15) destiné à être en contact mécanique avec la partie (11) et adapté pour détecter les vibrations et pour produire un signal primaire (23) électrique ou hertzien représentatif des vibrations, et
- une unité de traitement local (13) adaptée pour recevoir le signal primaire (23), pour extraire du signal primaire (23) au moins une information représentative d'une durée d'utilisation du dispositif médical (7) par le patient, et pour produire au moins un signal secondaire (29) contenant ladite information, le signal secondaire (29) étant destiné à être envoyé à au moins un serveur distant (3) adapté pour recevoir le signal secondaire (29) et pour extraire ladite information du signal secondaire (29).

2. Dispositif (9 ; 100) selon la revendication 1, dans lequel l'information extraite est représentative d'une durée d'utilisation du dispositif médical (7) par le patient dans un intervalle de temps, par exemple de 24 heures.

3. Dispositif (9 ; 100) selon la revendication 1 ou 2, dans lequel le capteur (15) comprend au moins une jauge à effet piézoélectrique (19) adaptée pour transformer les vibrations en une tension électrique.

4. Dispositif (9; 100) selon l'une quelconque des revendications 1 à 3, comprenant en outre au moins une liaison filaire (17) reliant le capteur (15) à l'unité de traitement local (13) et adaptée pour véhiculer le signal primaire (23) du capteur vers l'unité de traitement local (13).

5. Dispositif (9 ; 100) selon la revendication 4, dans lequel la liaison filaire (17) est adaptée pour alimenter le capteur (15) en énergie électrique en provenance de l'unité de traitement local (13).

6. Dispositif (9 ; 100) selon l'une quelconque des revendications 1 à 5, dans lequel l'unité de traitement local (13) comprend un émetteur hertzien (31) destiné à émettre le signal secondaire (29) vers le serveur distant (3).

7. Dispositif (9 ; 100) selon l'une quelconque des revendications 1 à 6, dans lequel :
- le capteur (15) comprend un émetteur (21) hertzien adapté pour émettre le signal primaire (23) vers l'unité de traitement local (13), et
- l'unité de traitement local (13) comprend un récepteur (26) hertzien pour recevoir le signal primaire (23).

8. Dispositif (9 ; 100) selon l'une quelconque des revendications 1 à 7, dans lequel l'unité de traitement local (13) comprend un module de traitement (27) adapté pour réaliser une analyse fréquentielle du signal primaire (23) sur des périodes de temps successives.

9. Dispositif (100) selon la revendication 8, dans lequel l'analyse fréquentielle comprend un calcul d'une transformée de Fourier sur chacune des périodes de temps sucessives.

10. Dispositif (100) selon la revendication 9, dans lequel le module de traitement (27) comprend une mémoire adaptée pour stocker :
- des valeurs (F1, F2) représentatives d'une fréquence minimale et d'une fréquence maximale à détecter par le module de traitement (27) dans la transformée de Fourier, ou
- des valeurs (A1, A2) représentatives d'un poids minimal et d'un poids maximal à détecter par le module de traitement (27) dans la transformée de Fourier, ou
- des valeurs (F3, F4) représentatives d'une gamme de fréquences, le module de traitement (27) étant adapté pour prendre en compte un pic de la transformée de Fourier uniquement si ce pic possède une largeur inférieure à la gamme de fréquences.

11. Dispositif (100) selon la revendications 10, comprenant au moins une prise de connexion (112), de préférence de type USB, destinée à permettre une connexion du dispositif avec un ordinateur propre à configurer le dispositif (100), de préférence propre à modifier lesdites valeurs (F1, F2, A1, A2, F3, F4).

12. Ensemble (5) comprenant :
- au moins un dispositif médical (7) comportant au moins une partie (11) animée de vibrations lorsque le dispositif médical (7) est utilisé par un patient pour recevoir un traitement médical, et
- un dispositif (9; 100) selon l'une quelconque des revendications 1 à 11, le capteur (15) étant en contact mécanique avec la partie (11) du dispositif médical (7).

13. Ensemble (5) selon la revendication 12, dans lequel le capteur (15) est fixé sur une surface de réception (24) de la partie (11), l'ensemble (15) comprenant au moins un ruban adhésif (25) collé, d'une part, sur le capteur (15) et, d'autre part, sur la surface de réception (24) de part et d'autre du capteur (15).

14. Installation (1) comportant au moins un ensemble (5) selon l'une quelconque des revendications 12 ou 13, et au moins un serveur distant (3) adapté pour recevoir le signal secondaire (29) et extraire ladite information du signal secondaire (29), le signal secondaire (29) étant envoyé au serveur distant (3).

15. Installation (1) selon la revendication 14, dans laquelle le dispositif (100) comprend une carte SIM (104) propre à permettre une identification du dispositif (100) par le serveur distant (3).
